# EUROPEAN PATENT APPLICATION

(11) **EP 4 039 277 A1**
(43) Date of publication of application: **10.08.2022**
(21) Application number: 21155755.8
(22) Date of filing: 08.02.2021
(51) Int. Cl.: A61K 47/00, A61K 9/00, G01N 33/543, C08G 65/22, C08G 65/326, C08G 65/333

(54) **LAYERED STRUCTURE FOR SPECIFIC CELL CAPTURING**

(71) Applicant: Freie Universität Berlin, 14195 Berlin (DE)
(72) Inventor: HAAG, Rainer, 12209 Berlin (DE); LEIXIAO, Yu, 12159 Berlin (DE); PENG, Tang, 12161 Berlin (DE)
(74) Representative: Maikowski & Ninnemann Patentanwälte Partnerschaft mbB

(57) **Abstract**

The present invention relates to a layered structure, comprising a) a base layer of nanoparticles of crosslinked molecules of a linear polyglycerol derivative, wherein the linear polyglycerol derivative has at least one first terminal catechol group; b) a coating layer comprising a hyperbranched polyglycerol derivative having at least one second terminal catechol group, wherein the hyperbranched polyglycerol derivative is covalently bound via the second terminal catechol group to the nanoparticles of the base layer; and c) a ligand layer comprising a ligand covalently bound to the hyperbranched polyglycerol derivative of the coating layer, wherein the ligand is capable of non-covalently bonding an antibody.

## Description

The present invention relates to a layered structure according to the preamble of claim 1, to a biointerface assembly comprising such a layered structure according to the preamble of claim 8, to a method for manufacturing such a layered structure according to the preamble of claim 10, to in vitro and in vivo uses according to the preambles of claims 11, 13 and 14, as well as to a method for capturing cells in vitro according to the preamble of claim 15.

It is well known that the extracellular matrix (ECM) is a complex fibrous network with micro/nano hierarchical features (ranging from 260 nm to 410 nm).^{[1]} Cell growth and functions are tightly associated with the cellular interactions at the cell-ECM biointerface.^{[2]} Over the past two decades, a series of micro/nano structured biointerfaces with the abilities to control cell adhesion/detachment and enhance cellular sensing to external signals have been developed.^{[2, 3]} These biointerfaces provide possibilities to mimic the natural nanostructure of ECMs and exhibit potential applications in tissue engineering and regenerative medicine.

The larger surface area and unique interactions between nanostructured biointerfaces and cells facilitate firm cellular attachment and exhibit great advantages in the isolation and detection of rare cells, e.g., the circulating tumor cells (CTCs).^{[4]} CTCs are cancer cells that detach from primary tumors or metastatic sites and circulate in the peripheral blood as the cellular origin of metastasis).^{[5]} Clinical researches indicated that the levels of CTCs in metastatic cancer patients is a predictor of survival.^{[6]} Cytogenetic evidence also matched aneusomic patterns between CTCs and those from the primary tumor.^{[7]} Enumeration and isolation of CTCs from patients' blood can provide insight into blood-borne metastasis, monitor disease progression, and help to guide the therapeutic outcomes of cancer.^{[8]} Therefore, technologies that can capture and detect CTCs directly from the patient's blood are highly desired from a clinical point of view for early cancer diagnosis. However, the concentration of CTCs in a patient's blood circulatory system is ultra-low, usually a few to hundreds per milliliter, therefore the isolation of CTCs from a patient's blood with high efficiency and purity is a big challenge.^{[9]}

In order to detect and isolate these rare CTCs, multiple isolation methods, e.g., microfluidic devices, immunomagnetic beads, and microfilter devices, have been developed by utilizing the differences among cellular physical properties or diversity of surface biomarkers of cancer cells)^{[0-13]} Among them, the methods based on affinity interaction between the bioligands at the interfaces and the biomarkers on cancer cell membrane yield higher efficiency and good specificity in contrast to mechanical and electrical sorting technique.^{[14]} But it is still difficult to isolate the CTCs with higher purity and viability.^{[15]}

Recently, increasing evidence indicates that biomimetic micro/nano topographic features at the cell-material interface could greatly improve the CTCs capture efficiency from patient blood.^{[16-19]} Compared to a flatter surface, the nanostructure features at the interface would provide more surface area for the functionalization of antibodies and/or aptamers, suitable sites for cancer cell trapping, as well as further lowering the rolling velocity of cells.^{[15]} Further results also demonstrate that the nanostructured features may induce the formation of pseudopodia on nanostructured surface^{[20]} and improve cell-material surface adhesion force,^{[21]} which further increases the affinity between cancer cells and nanostructured substrates. Thus, more and more attention has been paid to developing functionalized structured biointerfaces, e.g., nanopillars, nanowires, nanorods, for rare CTCs capture even if it generates another technological challenge to fabricate those nano/micro hierarchical substrates.^{[13, 22]}

While higher nonspecific proteins and cells (white blood cell) binding was also consequently observed on the rough nanostructured surfaces in the absence of specific bioligand^{[23]} and antifouling background.^{[24]}

It is an object of the present invention to provide a molecular structure being able to serve as cell capturing tool. It is another object of the present invention to provide methods and uses associated with such a molecular structure.

This object is achieved with a layered structure having the features of claim 1. Such a layered structure comprises a base layer, a coating layer and a ligand layer.

The base layer comprises or essentially consists of nanoparticles of crosslinked molecules of a linear polyglycerol derivative. In this context, the linear polyglycerol derivative bears at least one first terminal catechol group.

The coating layer comprises or essentially consists of a hyperbranched polyglycerol derivative bearing at least one second terminal catechol group. In this context, the hyperbranched polyglycerol derivative is covalently bound via the second terminal catechol group to the nanoparticles of the base layer.

The ligand layer comprises or essentially consists of a ligand is covalently bound to the hyperbranched polyglycerol derivative of the coating layer. In this context, the ligand is capable of non-covalently bonding an antibody.

This layered structure can also be denoted as advanced biointerface that integrates biospecific affinity interaction and well-defined topographical features for cell capturing such as cancer cell isolation. The layered structure serves as coating polymer that can be used to fabricate defined nanostructures via a simple dip-coating method with a definable surface roughness ranging from the nanometer to the sub-micrometer scale. Similar to previous reports about cell adhesion on rough surfaces,^{[27, 28]} the adhesion and isolation of cells, in particular of cancer cells, on this nanostructured biointerface exhibits an unique biphasic manner.

To achieve a high capture specificity, the coating layer comprising a bioinert hyperbranched polyglycerol (hPG) monolayer serves as a strong antifouling background to resist the nonspecific adhesion of proteins and blood cells (red blood cell, white blood cell, platelets, etc.).

In an embodiment, the chemical properties of hPG and the ligand were exploited to conjugate an epithelial-cell adhesion molecule antibody (anti-EpCAM) onto the biointerface via a revisable *cis*-diols-phenylboronic ester binding. The resulting nanostructure features greatly promoted the formation of cellular focal adhesion and filopodia in addition to the increase of surface area for antibody conjugation.

In an embodiment, the resulting biointerfaces that combine biospecific affinity interaction and surface nanostructures displayed an ultra-high capture efficiency for CTCs (>95%) with high purity (>97%) and sensitivity, even for cells with low EpCAM expression, e.g., the MDA-MB-231 and A549 cells.

In an embodiment, upon exposure to a glycan molecule having a higher affinity to phenylboronic acid, e.g., sorbitol,^{[29]} the captured CTCs were responsively released from the biointerfaces in a short time without damage due to the competitive binding.
In an embodiment, the base layer has a surface roughness lying in a range of from 1 nm to 1 µm, in particular of from 5 nm to 950 nm, in particular of from 10 nm to 900 nm, in particular of from 15 nm to 850 nm, in particular of from 20 nm to 800 nm, in particular of from 25 nm to 750 nm, in particular of from 30 nm to 700 nm, in particular of from 35 nm to 650 nm, in particular of from 40 nm to 600 mm, in particular of from 50 nm to 550 nm, in particular of from 75 nm to 500 nm, in particular of from 100 nm to 450 nm, in particular of from 150 nm to 400 nm, in particular of from 200 nm to 350 nm, in particular of from 250 nm to 300 nm.

The surface roughness as used herein corresponds, in an embodiment, to the root mean squared surface roughness Rq or RMS. It is typically 10 % higher than the arithmetical mean deviation of the assessed profile Ra. In an embodiment, the surface roughness, in particular Rq, is measured according to ISO 4287:1997.

In an embodiment, the degree of catecholization of the linear polyglycerol derivative lies in a range of from 10 % to 100 %, in particular of from 20 % to 90 %, in particular of from 30 % to 85 %, in particular of from 40 % to 80 %, in particular of from 50 % to 75 %, in particular of from 60 % to 70 %. In this context, the degree of catecholization indicates the amount of hydroxyl groups of the linear polyglycerol derivative that have been substituted by the first terminal catechol group.

In an embodiment, the degree of catecholization of the hyperbranched polyglycerol derivative lies in a range of from 1 % to 50 %, in particular of from 2 % to 40 %, in particular of from 3 % to 30 %, in particular of from 4 % to 20 %, in particular of from 5 % to 15 %, in particular of from 6 % to 14 %, in particular of from 7 % to 13 %, in particular of from 8 % to 12 %, in particular from 9 % to 11 %. In this context, the degree of catecholization indicates the amount of hydroxyl groups of the hyperbranched polyglycerol derivative that have been substituted by the second terminal catechol group.

In an embodiment, the linear polyglycerol derivative has a structure corresponding to general formula (I):

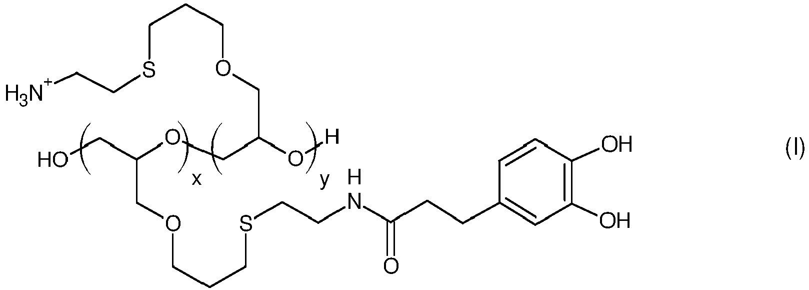

In this context, x is 1 to 1000, in particular 2 to 900, in particular 3 to 800, in particular 4 to 700, in particular 5 to 600, in particular 6 to 500, in particular 7 to 400, in particular 8 to 300, in particular 9 to 200, in particular 10 to 100, in particular 20 to 80, in particular 40 to 60. Furthermore, y is 1 to 1000, in particular 2 to 900, in particular 3 to 800, in particular 4 to 700, in particular 5 to 600, in particular 6 to 500, in particular 7 to 400, in particular 8 to 300, in particular 9 to 200, in particular 10 to 100, in particular 20 to 80, in particular 40 to 60.

In an embodiment, the ligand is covalently bond to the hyperbranched polyglycerol derivative via a phenylboronic ester bond. Such covalent bonding ensures a particularly strong connection between the ligand and the hyperbranched polyglycerol derivative.

In an embodiment, the ligand is a phenylboronic-acid modified biotin. Biotin is known to interact strongly with avidin, streptavidin, streptavidin derivatives or avidin derivatives. Thus, by covalent bonding the phenylboronic-acid modified biotin to the hyperbranched polyglycerol derivative layer, a universal binding partner for avidins can be introduced into the layered structure.

In an embodiment, the layered structure further comprises an antibody non-covalently bound to the ligand. Due to such a non-covalent bonding of the antibody, it is particularly easy to remove or replace the antibody by a different entity, e.g., a different antibody.

In an embodiment, the antibody is bound to the ligand via an avidin linker comprising or essentially consisting of avidin or at least one avidin derivative. Streptavidin or NeutrAvidin are particularly appropriate avidin derivatives that can be used to form the avidin linker.

In an aspect, the present invention relates to a biointerface assembly, comprising a substrate and a layered structure according to any of the preceding explanations. As explained above, such a layered structure has a base layer of nanoparticles of crosslinked molecules of a linear polyglycerol derivative. Furthermore, the linear polyglycerol derivative has at least one first terminal catechol group, wherein the nanoparticles of the layered structure are covalently bound to the substrate via the first terminal catechol group.

In an embodiment, the substrate comprises at least one of magnetic nanoparticles, glass, polycarbonate, poly(methyl methacrylate), and polystyrene. All of these substrates are particularly appropriate to receive the layered structure and thus to form together with the layered structure the biointerface assembly.

In an aspect, the present invention relates to method for manufacturing a layered structure according to the preceding explanations. This method comprises the steps explained in the following.

In a first step, a crosslinking and an aggregation of a linear polyglycerol derivative is allowed to take place in a basic aqueous buffer. In this context, the linear polyglycerol derivative has at least one first terminal catechol group, so as to form nanoparticles of the linear polyglycerol derivative. The nanoparticles formed by the cross-linking and aggregation process represent a base layer. This base layer is typically formed directly on a supporting surface.

In another step, a coating layer is applied onto the nanoparticles. In this context, the coating layer comprises a hyperbranched polyglycerol derivative having at least one second terminal catechol group. The hyperbranched polyglycerol derivative is covalently bound via the second terminal catechol group to the nanoparticles of the base layer.

In another step, a ligand layer is formed on top of the coating layer. In this context, the ligand layer comprises a ligand covalently bound to the hyperbranched polyglycerol derivative of the coating layer. The ligand is capable of non-covalently binding an antibody.

In an embodiment, the first step (i.e., the step of forming the base layer) is performed over a time period lying in a range of from 1 hour to 24 hours, in particular of from 1.5 hours to 20 hours, in particular of from 2 hours to 15 hours, in particular of from 2.5 hours to 12 hours, in particular of from 3 hours to 10 hours, in particular of from 3.5 hours to 8 hours, in particular of from 4 hours to 6 hours.

It turned out that the surface roughness of the base layer and thus the surface roughness of the whole layered structure mainly depends on the time used for the first method step. A particularly appropriate surface roughness for capturing cells, in particular cancer cells such as circulating tumor cells, could be obtained by performing the first method step over a time period of from 2 hours to 6 hours, in particular of from 3 hours to 5 hours, in particular of around 4 hours.

In an aspect, the present invention relates to a use of the layered structure according to the preceding explanations or of a biointerface assembly according to the preceding explanations for capturing cells in vitro.

In an embodiment, the cells to be captured in vitro are circulating tumor cells (CTCs).

In an aspect, the present invention relates to the medical use of the layered structure according to the preceding explanations or of the biointerface assembly according to the preceding explanations in in-vivo diagnostics or in therapy.

In an aspect, the present invention relates to the medical use of the layered structure according to the preceding explanations or of the biointerface assembly according to the preceding explanations in in-vivo diagnostics or therapy of a cancer disease. In an embodiment, the cancer disease is chosen from breast cancer, prostate cancer, lung cancer, pancreas cancer, colon cancer, connective tissue cancer, lymphoma, and leukemia.

In an aspect, the present invention relates to a method for capturing and optionally afterwards releasing cells in vitro. This method comprises the steps explained in the following.

In a first step, a layered structure according to any of the preceding explanations or a biointerface assembly according to any of the preceding explanations with such a layered structure is provided. In this context, the layered structure comprises a base layer, a coating layer covalently bonded to the base layer, and an antibody non-covalently bound to the ligand via an avidin linker.

In a further step, the layered structure is brought into contact with a body fluid sample. Blood and urine are particularly appropriate body fluids. This contact allows capturing of cells out of the body fluid sample by the antibody.

In a further optional step, the cells are released together with the bound antibody by rinsing the layered structure with a solution comprising a cis-1,2-diol group. The cis-1,2-diol group competes with the avidin linker against the binding partner entity of the ligand (e.g., a biotin entity). Particular appropriate compounds comprising a cis-1 ,2-diol group are sorbitol and other polyols (sugars) with such a cis-1,2-diol group.

In an embodiment, the step of releasing the previously captured cells takes place within a time period lying in a range of from 5 minutes to 60 minutes, in particular of from 10 minutes to 15 minutes, in particular of from 15 minutes to 45 minutes, in particular of from 20 minutes to 30 minutes. Typically, captured cells are not negatively influenced or harmed by a high osmotic pressure (resulting from an increased concentration of the compound comprising a cis-1,2-diol group) lasting on the cells over such a comparatively short time period.

With the methods being presently available, it is possible to count CTCs in the bloodstream (or in other body fluids). With the presently claimed method, it is possible to capture CTCs and other cells to allow a better characterization of the cells. To give an example, a polymerase chain reaction (PCR) can be performed after having captured and thus enriched the specific cell type of interest to further characterize these cells.

In an aspect, the present invention relates to a medical method for capturing and optionally afterwards releasing cells in vivo. This method comprises the steps explained in the following.

In a first step, a layered structure according to any of the preceding explanations or a biointerface assembly according to any of the preceding explanations with such a layered structure is administered to a patient in need thereof. In this context, the layered structure comprises a base layer, a coating layer covalently bonded to the base layer, and an antibody non-covalently bound to the ligand via an avidin linker.

In a further step, the layered structure is brought into contact with a body fluid of the sample. Blood and urine are particularly appropriate body fluids. This contact allows capturing of cells out of the body fluid by the antibody.

The captured cells can then be used for therapeutic or diagnostic purposes.

In a further optional method step, the layered structure is removed from the patient again. In doing so, it is possible to remove the captured cells from the patient's body fluid. Furthermore, such removal can facilitate subsequent therapeutic or diagnostic method steps.

In a further optional step (with or without prior removal of the layered structure from the patient), the cells are released together with the bound antibody by rinsing the layered structure with a solution comprising a cis-1,2-diol group such as sorbitol.

All embodiments of the layered structure can be combined in any desired way and can be transferred either individually or in any arbitrary combination to the biointerface assembly, to the different methods, and to the different uses. Likewise, all embodiments of the biointerface assembly can be combined in any desired way and can be transferred either individually or in any arbitrary combination to the layered structure, to the different methods, and to the different uses. Likewise, all embodiments of the individual methods can be combined in any desired way and can be transferred either individually or in any arbitrary combination to any of the other methods, to the layered structure, to the biointerface assembly, and to the different uses.

Finally, all embodiments of the individual uses can be combined in any desired way and can be transferred either individually or in any arbitrary combination to any of the other uses, to the layered structure, to the biointerface assembly, and to the different methods.

Further details of aspects of the present invention will be explained in the following with respect to exemplary embodiments and accompanying Figures. In the Figures:
- Figure 1: shows a scheme illustrating the manufacturing of a nanostructured multivalent biointerface for cell adhesion and isolation;
- Figures 2A to 2F: show different stages and test results of the fabrication of nanostructured surfaces via a layered structure;
- Figures 3A to 3D: show biospecific adhesion/capture of cancer cells on a layered structure in a roughness-selective manner;
- Figures 4A to 4D: show the performance of CTC capture on antibody-functionalized nanostructured mPG4 surfaces;
- Figures 5A to 5D: show further results elucidating the underlying mechanism for cancer cell capture on nanostructured surfaces in a surface-roughness selective manner; and
- Figures 6A to 6E: show results of experiments on the sorbitol-induced release of captured cancer cells from nanostructured surfaces.

The Figures will be explained in connection with an exemplary embodiment.

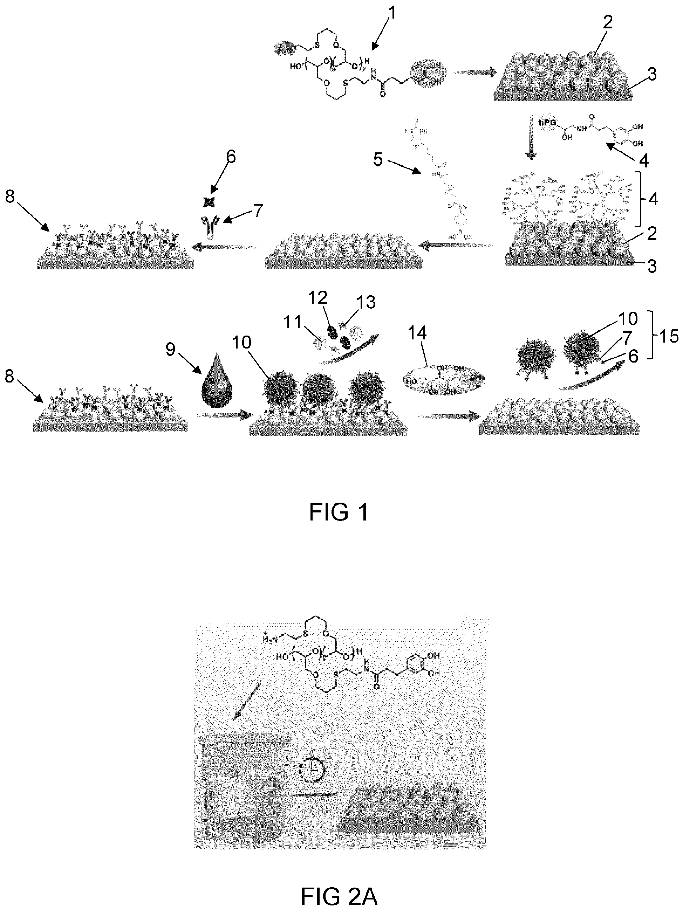

Figure 1 shows a general scheme on a method for manufacturing a layered structure in form of a nanostructured multivalent biointerface for specific cell adhesion/isolation, in particular CTC adhesion/isolation, and subsequent release. A mussel-inspired linear polyglycerol derivative 1 (mPG) is used as linear polyglycerol. Due to intermolecular aggregation and cross-linking, nanoparticles 2 of the linear polyglycerol derivative 1 are formed on a surface 3. Afterwards, a catecholic hyperbranched polyglycerol with 5 % of its free hydroxyl groups being substituted by a catechol group (i.e., having a degree of catecholization of 5 %) was applied as hyperbranched polyglycerol derivative 4 on the surface of the nanoparticles 2.

Subsequently, a phenylboronic-acid modified biotin 5 was applied on top of the hyperbranched polyglycerol derivative 4. Then NeutrAvidin 6 and an anti-EpCAM antibody 7 were applied on top of the nanoparticles 2 representing a layered structure. A biointerface assembly 8 resulted.

If a blood sample 9 comprising circulating tumor cells 10, white blood cells 11, red blood cells 12, and further cells 13 is then brought into contact with the biointerface assembly 8, only the circulating tumor cells will be captured by the anti-EpCAM antibody 7 bound to the nanoparticles 2. Thus, it is possible to use the biointerface assembly 8 to specifically capture circulating tumor cells 10. Furthermore, it is possible to release these captured circulating tumor cells 10 by adding sorbitol 14 to the biointerface assembly 8 with the captured circulating tumor cells 10. The sorbitol 14 effects a release of a conjugate 15 comprising NeutrAvidin 6, the anti-EpCAM antibody 7 and the captured circulating tumor cell 10.

The manufacturing process will be explained in more detail in the following.

### Materials

All chemicals and solvents are reagent or HPLC grade, used as received and purchased from Sigma (Steinheim, Germany) unless stated otherwise. Dialysis was performed in benzoylated cellulose tubes from Sigma-Aldrich (D7884, width: 32 mm, molecular weight cut-off (MWCO) 2000 g·mol-1). The deionized water used was purified using a Millipore water purification system with a minimum resistivity of 18.0 MΩ·cm. The hyperbranched polyglycerol with Mn = 5000 g·mol⁻¹ and Mw = 7500 g·mol⁻¹, was polymerized by a one-step ring-opening anionic polymerization (ROAP) as described in the literature.^{[45,46]} The trimethylolpropane (TMP) was used as the initiator.

### Synthesis of mPG coating polymer

The synthesis of the linear polyglycerol derivative (mPG coating polymer) was carried out according to the following reaction scheme and the subsequent explanations:

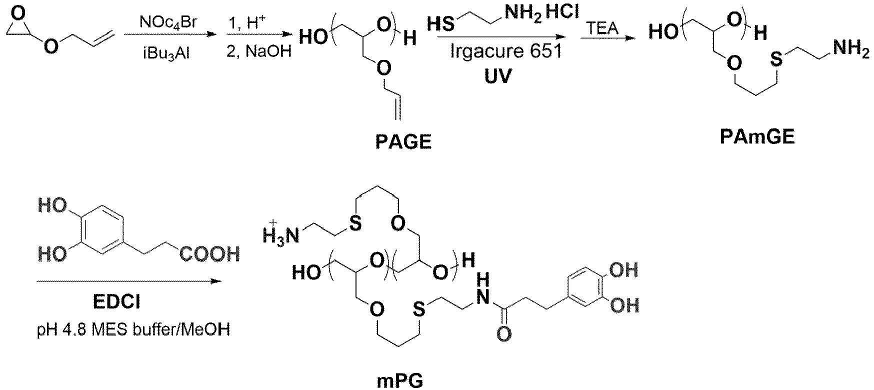

### Poly (allyl glycidyl ether) (PAGE)

Tetraoctylammonium bromide (546 mg, 1 mmol) was heated to melting temperature under vacuum to remove the trace water. After cooling down to room temperature, 140 mL abs. toluene was added into the Schlenk flask under argon to dissolve this initiator. Then 5.203 mL allyl glycidyl ether (AGE, 5.006 g, 44 mmol) were added into the solution. The polymerization was started by adding the triisobutylaluminum (3.6 mL, 4 mmol) at a constant temperature of 0 °C. The reaction mixture was allowed to warm up to room temperature and stirred overnight. After polymerization, 0.5 mL water was added to quench the polymerization and then the mixture was dried with Na₂SO₄. A colorless polymer was obtained after the removal of toluene under reduced pressure. Then the polymer was dissolved into Et₂O and centrifuged to remove the residues of initiator and catalyst. The obtained sticky polymer was re-dissolved in tetrahydrofuran (THF) and stirred overnight at room temperature followed by adding 2 mL 1 M NaOH aqueous solution. After removal of the salt and the solvent, the product was further purified by dialysis in dichloromethane and a kind of honey-like compound was obtained. ¹H-NMR (400 MHz, chloroform-d) δ = 5.96 - 5.8 (m, 1H, CH₂OCH₂C*H*=CH₂), 5.32 - 5.09 (m, 2H, CH₂OCH₂CH=C*H*₂), 3.97 (d, 2H, CH₂OC*H*₂CH=CH₂), 3.80 - 3.38 (m, 7H, PG-backbone). *M*_{n,GPC,THF} = 5796 g/mol, *Mw*/*Mₙ* = 1.09.

### Thiol-ene reaction (poly (amino glycidyl ether), PAmGE)

The thiol-ene reactions were carried out by UV irradiation (-1.2 mW/cm², λ = 365 nm) under ambient laboratory conditions. 1 g PAGE and cysteamine hydrochloride (3.98g, 4 eqv. to allyl groups) were dissolved into 40 mL methanol/THF (1:1). Then 2, 2-dimethoxy-2-phenylacetophenone (Irgacure 651, 2% eqv. to allyl groups) was added and the reaction was conducted under the irradiation of 365 nm UV light at room temperature. The reaction was monitored via ¹H-NMR and completed in 4 hours. Then, 5 mL triethylamine (Et₃N) was added into the mixture and stirred at room temperature for 10 hours to remove the hydrochloride. The formed triethylamine hydrochloride salts were filtrated and the filtrate was purified by dialysis in methanol.
¹H-NMR (400 MHz, MeOD-d4) δ = 1.78-1.95 (O-CH₂-C***H***₂-CH₂-S, 2H), 2.60-2.73 (O-CH₂-CH₂-C***H***₂-S, 2H), 2.77-2.9 (S-C***H***₂-CH₂-N, 2H), 3.11-3.20 (S-CH₂-C***H***₂-N, 2H), 3.8-3.43 (PG backbone), (O-C***H***₂-CH₂-, 2H).

### Catechol coupling (mPG)

The mussel-inspired coating polymer was prepared by the amide-coupling reaction between the PAmGE polymer and the 3,4-dihdroxyhydrocinnamic acid (DHHA). To prevent the Michael addition and dopa-dopa coupling during the reaction, an acidic buffer was used and the grafting density of catechol groups was precisely controlled by the equivalence ratio of amino groups to carboxyl groups. PAmGE and 1-Ethyl-3-(3-dimethylaminopropyl) carbodiimide hydrochloride (EDCI, 3 eqv. to amino groups) were dissolved into pH 4.8 MES aqueous buffer/methanol (1/1, v/v) mixture solvent. Then 3,4-dihdroxyhydrocinnamic acid (3 eqv. to amino groups) was added into the mixture and gently stirred overnight at room temperature. The product was purified by dialysis and the grafting density of catechol groups was calculated by the ¹H-NMR, which was 70%.
¹H-NMR (400 MHz, MeOD-d4) δ = 1.67-1.95 (O-CH₂-C***H***₂-CH₂-S, 2H), 2.34-2.68 (O-CH₂-CH₂-C***H***₂-S, 2H), (S-C***H***₂-CH₂-N, 2H), (CONH-C***H***₂-CH₂-Ar, 2H); 2.69-2.94 (S-C***H***₂-CH₂-N, 2H), (CONH-CH₂-C***H***₂-Ar, 2H); 3.8-3.43 (PG backbone), (O-C***H***₂-CH₂-, 2H).

### Synthesis of hPG-Cat5 polymer

The synthesis of the hyperbranched polyglycerol derivative (hPG-Cat5 polymer) was carried out according to the following reaction scheme and the subsequent explanations:

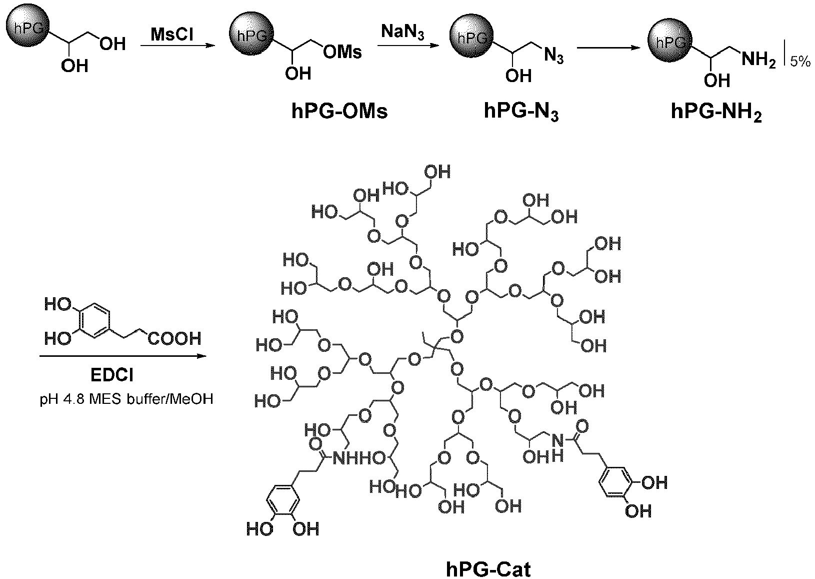

### O-Mesylpolyglycerol (hPG-OMs)

The reaction was carried out under argon atmosphere and exclusion of water. Hyperbranched polyglycerol (10.0 g, 135 mmol OH-groups) was dissolved in abs. dimethylformamide (DMF, 50 mL) in a Schlenk flask and cooled down to 0 °C in an ice bath. A solution of MsCI (8.1 mmol) in abs. DMF (10 mL) was added dropwise into the cooled hPG solution. After stirring under argon atmosphere for 16 h, the solvent was removed by rotary evaporation, and the residue was re-dissolved and dialyzed in MeOH for 24 h to give a brown honey-like product.
¹H-NMR (400 MHz; D₂O): δ = 4.14-3.39 (m, PG-backbone); and 3.32-3.28 (s, Ms); 1.43-1.41 (m, CC**H**₂CH₃ of starter); 0.89 (t, CCH₂C**H**₃, of starter) ppm.

### Polyglycerolazide (hPG-N3)

O-mesylpolyglycerol was dissolved in DMF upon ultra-sonification in a flask with a reflux condenser. After the addition of NaN₃ (3 eq. to -OMs groups), the resulting suspension was heated to 80 °C for 3 days. After cooling down to room temperature, the mixture was filtrated by celite to remove away the excess NaN₃. The filtrate was concentrated and the residue was further purified by dialysis in H₂O for 24 h to give a brown paste-like compound.
¹H-NMR (400 MHz; MeOD): δ = 4.2-3.45 (m, PG-backbone); 1.43-1.41 (m, CC**H**₂CH₃ of starter); 0.89 (t, CCH₂C**H**₃, of starter) ppm. IR: v (cm⁻¹): 3365, 2869, 2096 (N₃), 1648, 1453, 1272, 1066, 930, 866.

### Polyglycerolamine (hPG-NH2)

Polyglycerolamine was synthesized by a Staudinger reaction. Polyglycerolazide and 1.2 eq of PPh₃ were dissolved in a mixture of THF/H₂O (3/1, v/v). After 2 days of reacting at 45 °C, THF in the mixture was removed by evaporation. The byproduct PPh₃O, which cannot be dissolved in H₂O, were removed by filtration. And the filtrate was then purified by dialysis in MeOH for 20h.
¹H-NMR (400 MHz; D₂O): δ = 4.12-3.4 (m, PG-backbone); 1.41-1.39 (m, 2H, CC**H**₂CH₃ of starter); 0.90 (t, 3 H, CCH₂C**H**₃, of starter) ppm. IR: v (cm⁻¹): 3349, 2864, 1659, 1597, 1456, 1322, 1069, 1024, 930, 863.

### Catecholic polyglycerol (hPG-Cat5)

The polyglycerolamine (with 5% amino groups) and 1-Ethyl-3-(3-dimethylaminopropyl) carbodiimide hydrochloride (EDCI, 3 eqv. to amino groups) were dissolved into pH 4.8 MES aqueous buffer/methanol (1/1, v/v) mixture solvent. Then 3,4-dihdroxyhydrocinnamic acid (3 eqv. to amino groups) was added into the mixture and gently stirred overnight at room temperature. The product was purified by dialysis and the grafting density of catechol groups was calculated by the ¹H-NMR.
¹H-NMR (400 MHz; MeOD): δ = 6.8-6.54 (Ar-H, 3H from catechol group.); 4.0-3.40 (PG backbone); 2.76 (2H, COC**H**₂CH₂C); 2.47 (2H, COCH₂C**H**₂C); 1.41-1.39 (2H, CCH₂CH₃ of starter); 0.90 (3 H, CCH₂C**H**₃, of starter) ppm.

### Biotin-phenylboronic acid bioligand (Biotin-PBA)

The synthesis of the ligand (Biotin-PBA) was carried out according to the following reaction scheme and the subsequent explanations:

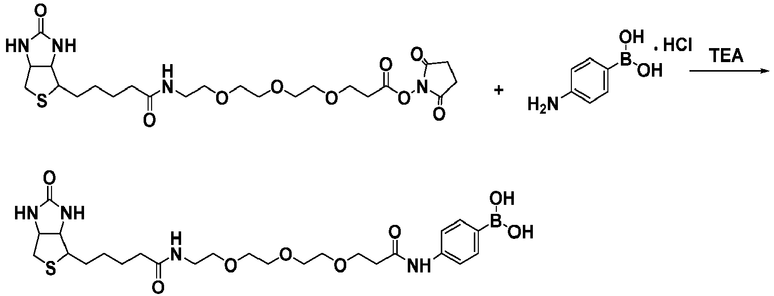

N-Succinimidyl 9-(biotinamido)-4,7-dioxanonanoate (Biotin-EG₂-NHS) (50 mg, 0.1 mmol) and 4-aminophenylboronic acid hydrochloride (16.5 mg, 0.095 mmol) were dissolved in abs. dry DMF in a 10 mL flask. Then 20 µL Et₃N was added and the mixture was stirred at room temperature for 4 h. After the reaction, the solvent was removed under high vacuum and the product was further purified via HPLC.

### Mussel-inspired mPG coating

Nanostructured mPG coatings were prepared via a dip-coating method. Generally, freshly cleaned glass slides were immersed into a solution of 1 mg/mL catecholic polyglycerol in a mixture of MeOH and 3-(N-morpholino)-propanesulfonic acid (MOPS, 0.1M, pH 8.6) buffer (v/v 3:1) at room temperature for 20 hours. The depth of the solution was 1 cm. After that, the coated slides were thoroughly rinsed with methanol and Milli-Q water, dried by N2 stream, and fully dried in vacuum.

### hPG-Cat coating

The prepared mPG coating slides were dipped into the coating solution of hPG-Cat5 (1mg/mL) in pH 8.6 MOPS buffer (0.1 M) at room temperature for 12 h. Under basic conditions, the catechol groups from hPG-Cat were oxidized into quinone and further reacted with the present amino groups from mPG coating via the Michael addition or coupled with catechol groups on the mPG surface via the dopa-quinone coupling. After that, the slides were thoroughly rinsed with methanol, water, and then dried by an N2 stream.

### Bioligands immobilization via the diols-boronic ester dynamic bonding

Phenylboronic acid-functionalized biotin was immobilized onto hPG-Cat coated mPG surface via the dynamic diols-boronic ester bonding. We immersed the hPG-mPG coating surface into PBA-Biotin solution (1 mM, in methanol) and kept them at room temperature for 4 hours. Then the functionalized slides were taken out and rinsed with methanol and water to remove unboned biotin-PBA, and then dried with an N2 stream.

### Biomarkers conjugation

The immobilization of anti-EpCAM onto biotin-mPG coating was realized via the high biospecific biotin-avidin noncovalent interaction. The biotin-mPG coating surfaces were incubated into 1 mg/mL NeutrAvidin (Thermo Fisher Scientific, USA) solution in DPBS at 37 °C for 1 h and then thoroughly rinsed with DPBS to remove excess NeutrAvidin. The obtained NeutrAvidin-mPG surface was kept in DPBS solution at 4 °C until ready for use. Before cell experiments, biotinylated anti-EpCAM (12.5 µg/mL in DPBS, containing 1 wt% BSA) were readily conjugated onto NeutrAvidin-mPG surfaces at 37 °C (incubation time: 1 h).

### Quartz Crystal Microbalance (QCM) with Dissipation

Quartz crystal microbalance (QCM, Q-Sense E1, Sweden) with dissipation was used to study the adsorption on the surfaces. QCM with dissipation monitors the changes in resonance frequency (Δf) and dissipation (ΔD) of a piezoelectric quartz crystal as a function of time. The f and D were recorded at the fundamental frequency (4.95 MHz) and its 3rd, 5th, 7th, 9th, 11th, and 13th overtones. Only the 3rd overtone was shown in the sensorgrams.

For the online coating of hPG-Cat5, the mPG4 coating sensor chip was inserted into the flow chamber (QFM 401, QSense, Sweden, internal volume of 40 µL) and incubated in pH 8.5 MOPS buffer with a flow rate of 0.1 mL/min at 25 °C until the equilibration of the baseline. A solution of hPG-Cat5 (1 mg/mL in pH 8.6 MOPS buffer) was pumped into the flow chamber (0.1 mL/min). After 1 h of dynamic online adsorption, the flow chamber was alternately rinsed with pH 8.6 MOPS buffer and Milli-Q water (0.1 mL/min). The Sauerbrey equation was used to calculate the mass of the adsorbates (Δm = C × Δf, where Δm is the change in mass, C is the mass sensitivity constant of the quartz crystal (-17.7 ng·cm⁻². Hz⁻¹), and Δf is the overtone-normalized frequency change).

The *in-situ* immobilization of the PBA-biotin onto the coating surface was also monitored by QCM. hPG-Cat5 coated mPG4 coating sensors were incubated in methanol. After baseline equilibrium, the PBA-biotin in methanol (1mM) was pumped into the flow chamber. After 30 min, the sensor was rinsed with DPBS buffer again until the baseline equilibrated again.

A similar protocol was used to study the conjugation of antibodies onto the coating surface. Biotinylated mPG coating sensors were incubated in pH 7.4 DPBS buffer. After baseline equilibration, the NeutrAvidin (1 mg/mL in DPBS buffer) was pumped into the flow chamber. After 30 min, the sensor was rinsed with DPBS buffer for 15 min. Then freshly prepared antibody solution (anti-EpCAM, 12.5 µg/mL in DPBS buffer with 1 wt % BSA) flowed into the NeutrAvidin modified sensor surface for 30 min. The sensor was rinsed with DPBS buffer again for another 15 min. The flow rate used for all experiments was 0.05 mL/min, and the temperature was 25 °C. The Voigt model for viscoelastic layers was used to calculate the mass of adsorbed proteins with the same method as we reported.

### Water Contact Angle (WCA)

Water contact angle was measured using a contact angle goniometer system (DataPhysics Instruments, Germany). A droplet of 2 µL Milli-Q water was placed on the substrate with the sessile drop method and equilibrated for 10 s at room temperature. Fifteen parts of five parallel coating slides were measured and averaged to get a reliable value.

### Atomic Force Microscopy (AFM)

The AFM results were recorded by a MultiMode Nanoscope V scanning probe microscopy (SPM) system (Bruker, USA) in the air to investigate the TiO2 surface and its coating with polymer. The commercially available AFM cantilever tips (PPP-NCLR-20 probes from NanoAndMore,GmbH) with a force constant of ∼ 48 N/m and resonance vibration frequency of ∼ 330 kHz were used, and the scanning rate was set at 0.8 Hz. The AFM mode, Peak Force QNM, was used for better controlling the force with which the tip interacts with the surface.

### Scanning electron microscope (SEM) for coating morphology

The surface morphology of the coatings was investigated with scanning electron microscope (SEM, Hitachi SU8030, Japan) at an accelerating voltage (Vacc) of 20 kV, a current of 10 µA and a working distance (WD) of around 8.3 mm. The samples were dried under high vacuum and coated with an 8-10 nm gold layer by using a sputter coater (Emscope SC 500, Quorum Technologies, UK) for 20 s at 30 mA, 10⁻¹ Torr (1.3 mbar) in an argon atmosphere.

### SEM images for cells on the mPG coating surface

After 3h culturing on mPG coating surface in RPMI 1640 medium, the cells were gently rinsed with DPBS, fixed with glutaraldehyde solution (2.5 wt % in DPBS) for 60 min at room temperature, and thoroughly removed the glutaraldehyde with DPBS. Cells were then dehydrated by mixtures of ethanol and DPBS with different ratios (30/70, 50/50, 70/30, 85/15, 95/5, 100/0, and 30 min for each) at room temperature. Residue water in cells was further thoroughly removed by hexamethyldisilazane/ethanol (50/50, v/v, 30min) and hexamethyldisilazane (30 min). After pipetting out hexamethyldisilazane, the coating slides with cells were put into a hood overnight at room temperature and then a desiccator in vacuum for drying. All samples were sputtered a thin gold layer (sputtering for 60 s, Emscope SC 500, Quorum Technologies, UK) and imaged with scanning electron microscope (Hitachi SU8030, high voltage on 10 kV).

### Cell Culture

Roswell Park Memorial Institute RPMI-1640 culture medium (Gibco) was supplemented with 10% (v/v) fetal bovine serum (FBS) (Gibco), and 1% (v/v) of penicillin-streptomycin solution (Gibco). Human breast cancer cell lines (MCF7, T47D, MDA-MB-231), human lung cancer cell line(A549) (DSMZ no.: 115), and human cervical cancer cell lines (HeLa) (DSMZ no.: ACC 57) were obtained from the American Type Culture Collection (ATCC). GFP⁺-HeLa cells were derived from the HeLa cell line (ATCC; CCI-2), which were stably transfected with an expression unit for a destabilized enhanced green fluorescent protein (d2EGFP, Clontech, Palo Alto, USA). All the cancer cell lines were routinely cultured at 37 °C with 5% CO₂ in a 24-well plate in an air atmosphere.

### Cancer Cell Capture

Before the capture experiment, the cancer cells were pre-stained with CellTraceTM violet dye (Thermo Fisher Scientific, Waltham, MA, USA) for 20 min by using the standard protocol. Stained cells were spiked at a density of 3000 cells/mL on anti-EpCAM functionalized mPG coating surface in serum-free RPMI-1640 culture medium at a concentration of 3000 cells/mL and incubated at 37 □ for 60 min. Then the mPG surfaces were gently rinsed with DPBS for three times and observed by the fluorescence microscope (Zeiss Axio Observer Z1, Germany). The corresponding capture efficiency was calculated via the previous reported method.^{[14]}

### Cancer cell capture from artificial patient blood samples

Human blood samples were collected in Vacutainer tubes (Becton Dickinson, Franklin Lakes, NJ, USA) from healthy donors. The blood samples were processed or lysed within 2 h after the blood draw. Briefly, the blood was lysed using red blood cell (RBC) lysing buffer (1 g potassium hydrogen carbonate, 18.3 g ammonium chloride, and 0.3 g anticoagulant ethylenediaminetetraacetic acid in 1 L H₂O, pH = 7.5). Different numbers of CellTraceTM violet pre-stained cancer cell lines were then spiked in healthy whole blood or lysed blood to prepare artificial patient blood samples. After incubated in the artificial patient blood samples for 60 min at 37 □, antibody-functionalized mPG surfaces were rinsed with DPBS and observed by fluorescence microscope (Zeiss Axio Observer Z1, Germany).

### Cell release and re-culture

Due to the dynamic diols-boronic ester bonding, the captured cancer cells on mPG surfaces can be released by adding D-sorbitol. The released cells were collected and seeded in a new cell culture plate for re-culturing. Cell Counting Kit-8 (CCK8) (Dojindo Molecular Technologies, Inc., Rockville, USA) assays were performed to test the proliferation of released cells and determine cell numbers based upon reaction product optical density (OD, at 450 nm) value. Microscope (Zeiss Axio Observer Z1, Germany) was used to observe and image the cells.

### Immunofluorescence staining

Cells were washed twice with DPBS before fixation with 4% paraformaldehyde at room temperature for 15 minutes. Samples were then washed 3 times with ice-cold PBS. Cells were permeabilized with 0.25% (v/v) Triton-X 100 in PBS for 10 minutes at room temperature, then washed 3 times with PBS. Non-specific antibody binding was blocked by incubating samples with 1% (w/v) bovine serum albumin (BSA) in PBST (0.1% v/v Triton-X 100 in PBS) at room temperature for 45 minutes. Next, samples were washed briefly with PBST and incubated with dye-labeled primary antibodies overnight at 4 °C. Following primary antibody incubation, samples were washed twice with PBST and 3 times with PBS. Samples were then incubated with DAPI for 60 minutes at room temperature for one hour, followed by washing twice with PBST and 3 times with PBS. Immunofluorescence images were acquired on a Zeiss Axio Observer Z1 microscope or a Leica SP8 confocal microscope.

### Mussel-inspired polymer coating with well-defined surficial features

To mimic the adhesion properties of mussel foot proteins, previously a mussel-inspired dendritic polyglycerol (MI-dPG) coating polymer was developed, and versatile coating surfaces have been successively fabricated via this building block.^{[27, 28, 30, 31]} Due to the multivalent character of the dendritic polyglycerol, most of the functional groups are exposed to the periphery.^{[30]} The crosslinking of the polymers is greatly accelerated, but the resulting roughness and topographic features of the coating are far beyond control.

To circumvent these limitations, aspects of the present invention relate to a new generation of mussel-inspired polyglycerol-based coating polymer (mPG) with a linear polymer architecture. Compared with the dendritic architecture, the linear mPG has a higher hydrodynamic radius and more entanglement conformation. Therefore, the reactivity of the heteromultivalent catechol groups and amine groups on the polymer chain was decelerated and the coating process was more controllable. The average molecular weight of the mPG coating polymer is about 10 kDa, which is similar to the mfp-5 (∼ 9kDa).^{[32]}

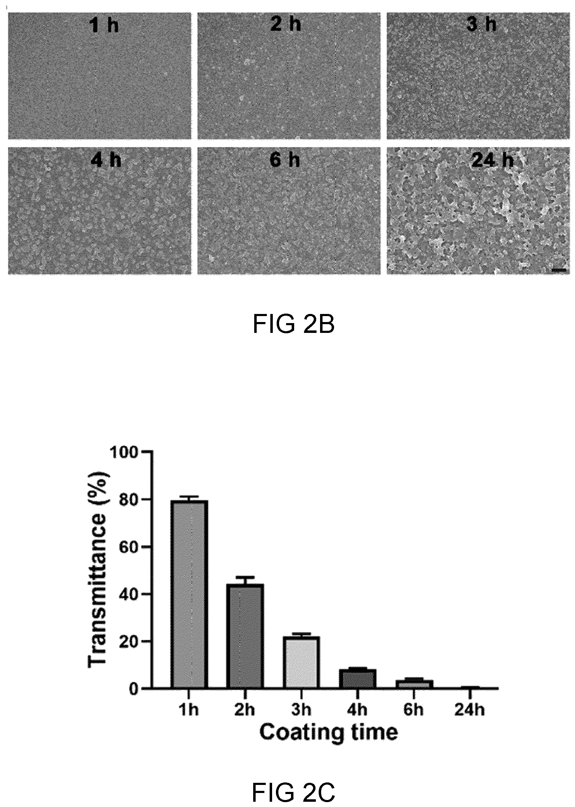

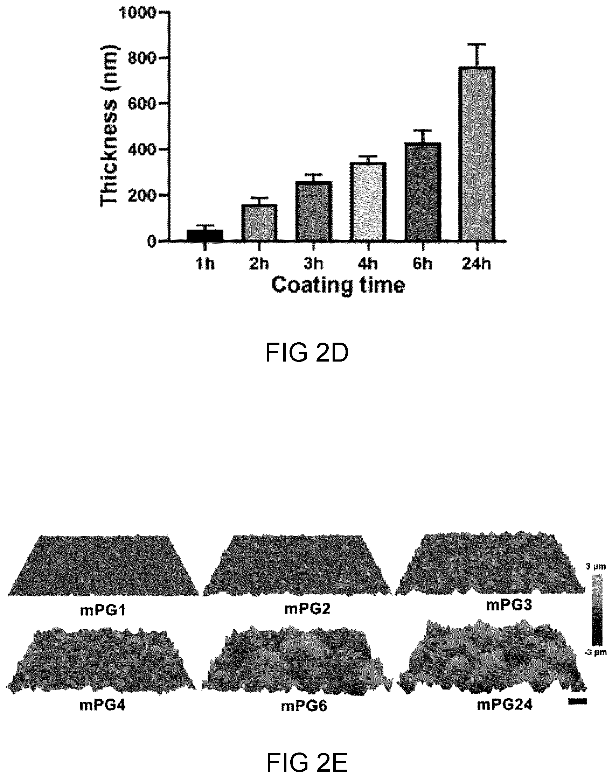

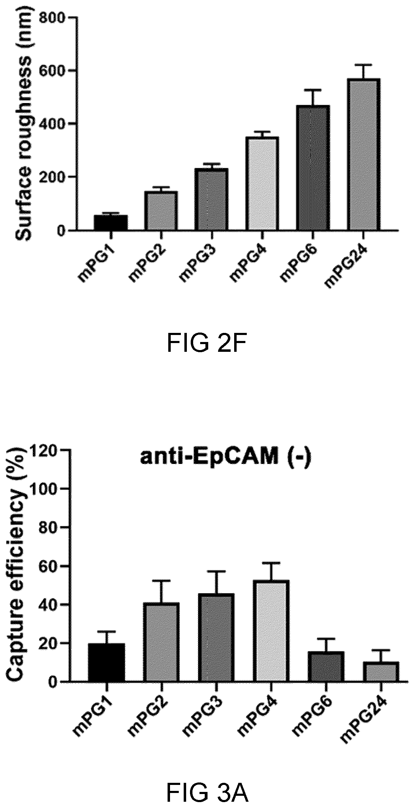

Figure 2A shows a one-step dip-coating method that was used to prepare surfaces with well-defined topographic features that result from the self-polymerization of mPG polymers under the basic condition and the deposition of formed nanoparticles from mixing solution. Figure 2AB shows scanning electron microscope images of the resulting mPG coating surfaces with the variation of dip-coating time. The scale bar indicates 10 µm. Time-dependent optical transmittance of the mPG coating surface is shown in Figure 2C and thickness of the mPG coating surface is shown in Figure 2D. Figure 2E shows the surface morphology and Figure 2F the corresponding surface roughness of mPG coating surfaces characterized by AFM. Scale bar indicates 10 µm. The mPG1, mPG2, mPG3, mPG4, mPG6, and mPG24 indicate the resulting mPG coatings with 1 hour, 2 hours, 3 hours, 4 hours, 6 hours, and 24 hours dip-coating time, respectively.

The functionality of the catechol groups in the mPG polymer (70 mol%) is ∼ 2.5 times higher than that in mpf-5 (27 mol%^{[33]}) and serves for for rapid deposition. Under basic conditions, the mPG underwent covalent crosslinking and aggregated into nanoparticles, which were partially insoluble in the coating solution (a mixture of methanol and water) and were deposited onto the substrate (Figure 2A). Furthermore, the deposited particles progressively grew from nanoscale to microscale due to the consecutive Michael addition and/or dopa-quinone coupling (Figure 2B). With the increase of incubation time at room temperature, the optical transmittance of the resulting coating surface was progressively decreased (Figure 2C). The thickness of mPG coating on the substrate continually increased with incubation and reached 340 nm after 4 hours and 760 nm after 24 hours (Figure 2D), which is quite slow compared with its dendritic analog^{[30]} but much faster than the crosslinking of dopamine under basic^{[34]} and even oxidative conditions.^{[35]} The surface morphology and root mean square roughness (Rq) of the resulting coating with different incubation time were further characterized by atomic force microscopy (AFM) (Figures 2E and 2F). mPG coating polymers adsorbed and gradually polymerized on the substrate surface and generated well-defined topographical features ranging from nanometers to micrometers with an increase of coating time (Figure 2E). The surface roughness of the resulting surfaces slowly increased from 56 nm (mPG1: 1-hour incubation) to 571 nm (mPG24: 24-hour incubation) (Figure 2F), which suggests that the inter/intra-polymer coupling reactions between catechol groups and amine groups are greatly affected by the polymer architecture. Thus, the resulting coating process could be performed in a more controllable manner than processes known from prior art.

### Cellular response to nanostructured mPG surfaces displayed a roughness selective manner

To investigate cell adhesion on the nanostructured surfaces, human breast cancer cells (MCF7) were seeded and cultured for 3 hours.

Figure 3A shows cell capture on nanostructured mPG surfaces.

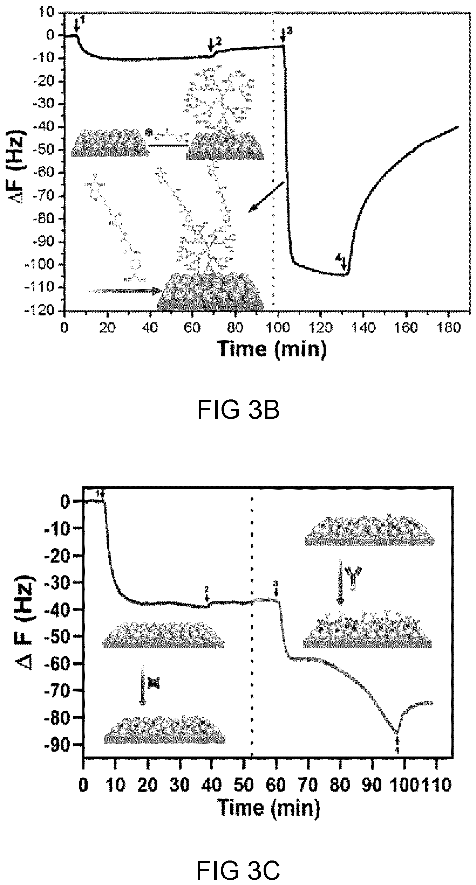

Figure 3B shows real-time quartz crystal microbalance (QCM) frequency shift of the adsorption of antifouling hPG-Cat polymers on mPG4 surface and the *in-situ* immobilization of bioligands onto the resulting hPG-mPG4 surface via the unique diol-boronic ester bonding between hPG and biotin-PBA. The arrow marked with 1 indicates the time point of an addition of hPG-Cat5 coating polymer. The arrow marked with 2 indicates the time point of an addition of buffer. The arrow marked with 3 indicates the time point of an addition of phenylboronic acid-biotin. The arrow marked with 4 indicates the time point of an addition of buffer.

Figure 3C shows real-time quartz crystal microbalance (QCM) frequency shift of the adsorption of NeutrAvidin and anti-EpCAM onto the biotin functionalized mPG4 surfaces via the bioorthogonal biotin-avidin interaction, respectively. The arrow marked with 1 indicates the time point of an addition of Neutravidin. The arrow marked with 2 indicates the time point of an addition of buffer. The arrow marked with 3 indicates the time point of an addition of an anti-EpCAM antibody. The arrow marked with 4 indicates the time point of an addition of buffer.

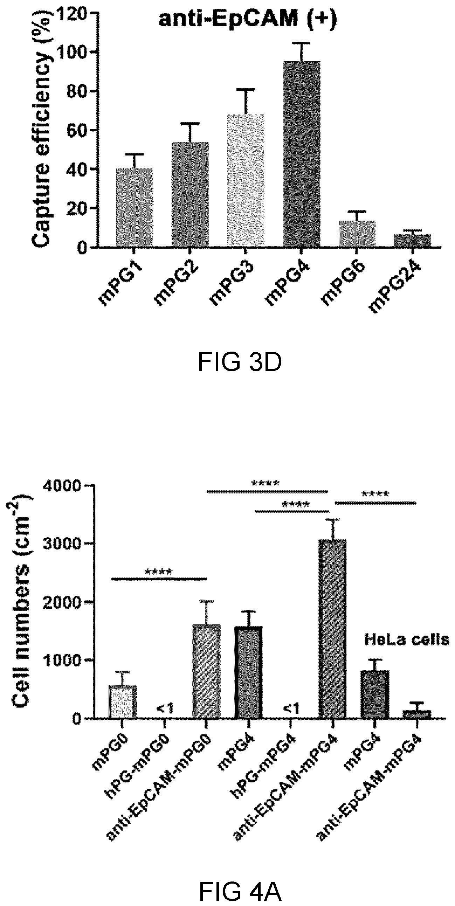

Figure 3D shows biospecific CTC capture efficiency on the antibody (anti-EpCAM) functionalized mPG coating surfaces.

As shown in Figure 3A, the adhesion of MCF7 cells on nanostructured mPG surfaces displayed an interesting biphasic manner. The corresponding cell capture efficiency gradually increased with the increase of surficial roughness and maximized on mPG4 surfaces (Rq = 352 nm, capture efficiency > 50%). Once the surface roughness was above 352 nm, the topographic features greatly restricted the cell adhesion, and thus the capture efficiency drastically reduced. This interesting cellular response to nanostructured surficial features agrees with previous reports on mesenchymal stem cells (MSC)^{[27, 28]} but the preferred roughness shifted to a lower region owing to the smaller size of cancer cells.

Based on this special cell adhesion behavior nanostructured surfaces, the inventors then asked whether these interfaces could be potentially developed for the capture and isolation of rare CTCs from blood samples. EpCAM (epithelial cell adhesion molecule) is a typical CTCs biomarker, which is overexpressed in most adenocarcinoma CTCs but negative-expressed in normal blood cellS.^{[36]} To obtain specific CTCs isolation, anti-EpCAM was then employed as a biospecific ligand and immobilized onto the nanostructured mPG coating surface. As mentioned above, a strong bioinert background is required to resist the interference of nonspecific proteins adsorption and blood cell (red blood cell, white blood cell, and platelets) adhesion during CTCs isolation. Therefore, a monolayer of antifouling hyperbranched polyglycerol (hPG) was introduced on the defined nanostructured biointerfaces to guarantee the high capture specificity and purity before bioligands conjugation.

Catechol functionalized hPG (5% functionality) quickly adsorbed onto the mPG4 coating surface and then coupled with the amino and/or quinone groups on the mPG4 surface to form a stable coating in the basic buffer (Figure 3B). Based on the quantification of QCM measurement, there are 88.4 ng/cm² of hPG-Cat polymers chemically anchored onto the mPG4 surface and generate a 1.37 nm monolayer coating on the mPG4h nanostructured surface. No obvious surface roughness change was observed after hPG grafting, which indicated the mPG4 surface still maintains its nanostructured features. In addition to the excellent antifouling properties, plenty of *cis*-diol groups on the hPG polymer also offer an opportunity to immobilize bioligands via the revisable *cis*-diol-phenylboronic ester bonding.

Thus, phenylboronic acid (PBA) modified biotin was grafted onto the hPG-mPG surfaces. The sharp decrease of frequency in real-time QCM curve indicated the rapid biotin binding on the hPG-mPG4 surface through the PBA/*cis*-diol esterification (Figure 3B). Subsequently, biotinylated anti-EpCAM antibody molecules were conjugated onto the coating surfaces via the biorthogonal avidin biolinker, which could form the strongest non-covalent bonding with biotin.^{[37]} 651.2 ng/cm² of NeutrAvidin and 667.1 ng/cm² of biotinylated anti-EpCAM were stepwise conjugated onto mPG4 surfaces to generate an anti-EpCAM functionalized biospecific nanostructured biointerfaces (Figure 3C).

Compared with the nanostructured mPG coating surface, the adhesion of MCF7 cancer cells (EpCAM positive cell line) on anti-EpCAM functionalized surfaces was significantly enhanced. The corresponding capture efficiency was greatly improved for the surface with lower roughness and maximized on the anti-EpCAM-mPG4 surface. More than 95% of spiked cells from the serum-free medium were captured onto antibody-functionalized mPG4 surface after 2 hours of incubation (Figure 3D). Thereafter, the capture efficiency on higher rough surfaces was drastically reduced. Furthermore, no cells were obviously observed on the hPG-mPG4 surfaces, indicating the nanostructured surfaces have an excellent bioinert background. To achieve the highly efficient capture of cancer cells, the nanostructured surfaces still required the functionalization of biospecific ligands to recognize the cells.

### High performance cancer cell capture on biospecific nanostructured surfaces

To assess the specific cancer cell capture on the anti-EpCAM-functionalized nanostructured interfaces, EpCAM-positive cancer cell line (MCF7^{[38]}) was used as the model cell line, whereas an EpCAM-negative cell line, namely, GFP⁺-Hela cells^{[39]}, and anti-EpCAM functionalized smooth surfaces were employed as negative control cell line and negative control surface, respectively.

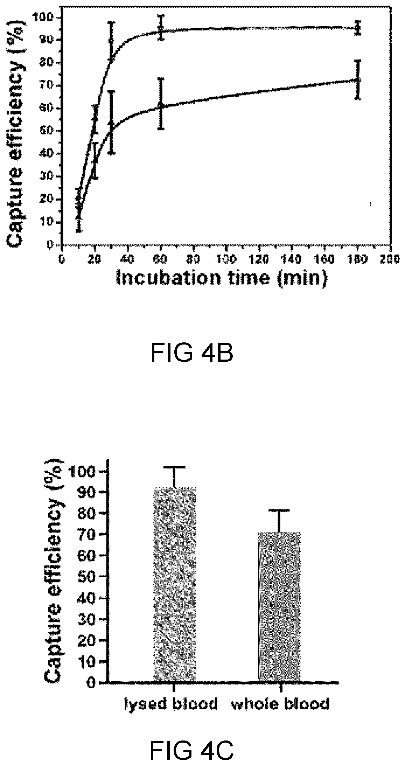

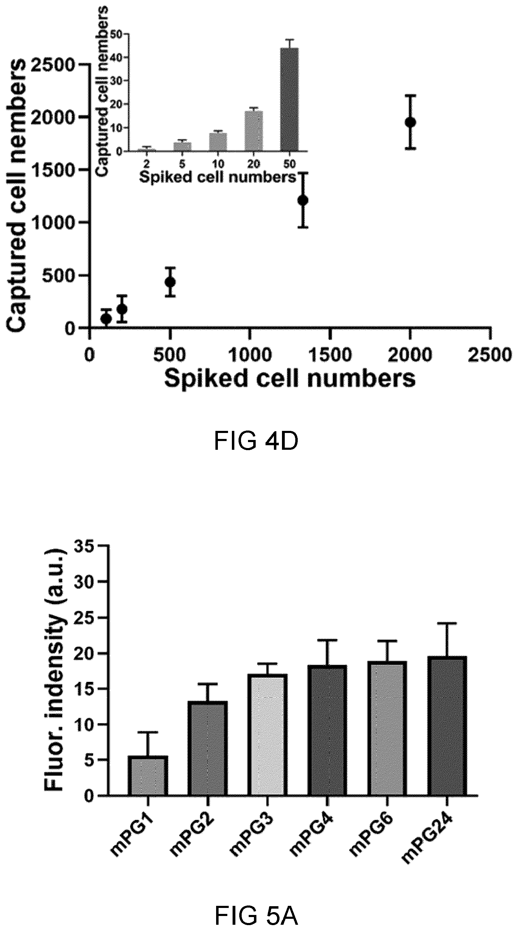

Figure 4A shows the number of cells captured on smooth mPG0 surface (pristine surface without mPG coating) and nanostructured mPG4 surfaces (n = 20-25, 3 technical replicates). Figure 4B shows time dependent cell capture on antibody functionalized smooth mPG0 surface and nanostructured mPG4 surfaces. Figure 4C shows biospecific cancer cell capture on anti-EpCAM-mPG4 surfaces from MCF7 spiked RBC lysed blood samples and whole blood samples (n = 20-25, 3 technical replicates). The spiked cell concentration was 2000 cells/mL. Figure 4D shows a linear correlation between the number of spiked and captured cells on anti-EpCAM-mPG4 surfaces from artificial patient blood samples (n = 3 for each condition). The inset indicates the capture performance for ultra-low concentrations of cancer cells (5, 10, 20, and 50 cells/mL of lysed blood, n = 6).

For microscopic observation, MCF7 cells were pre-stained with a fluorescent tracker dye. All experimented cells were spiked in serum-free RPMI-1640 culture medium with a concentration of 3000 cells/mL. Unless otherwise specified, the nanostructured biointerface in the following part was the antibody functionalized mPG4 surfaces with optimized antibody conjugation density. Benefiting from the nanostructured features, cancer cell capture efficiency on both mPG4 surfaces with and without antibody functionalization was greatly improved compared to the smooth control surfaces (Figure 4A). More than 95% of spiked cells adhered to the anti-EpCAM-mPG4 surface whereas only 53% of the spiked cells were captured onto the antibody functionalized smooth surfaces. Under the same condition, less than 5% of EpCAM-negative HeLa cells were observed on antibody functionalized mPG4 surfaces (Figure 4A). The present anti-EpCAM displayed a good biospecific property and remarkably reduced the nonspecific cell adhesion. Thus, only EpCAM positive MCF7 cells were selectively captured by this biointerface from the MCF7/HeLa mixture. The hierarchical nanostructured features provide more surface area for cell interaction with the biointerfaces and could further lower the rolling velocity of cells. Therefore, spiked cancer cells tend to interact with the nanostructure features and quickly adhered to the biointerface. The corresponding capture efficiency on nanostructured mPG4 surface reached to 90% after 30 min and maximized to 95% after 1-hour incubation, which is about two or three times faster than many reported biointerfaces for cancer cell capture.^{[40, 41]} For the smooth control surface, the capture efficiency gradually reached 65% even after incubation for 3 hours (Figure 4B). It suggests that the nanostructured features on the surfaces could significantly facilitate the adhesion of cancer cells on the biointerfaces with higher capture efficiency, higher bioselectivity, and shorter detection time, potentially being applied for fast cancer diagnosis. On the other hand, this significant enhancement of biospecificity and bioselectivity may also be ascribed to the excellent bioinert background generated from the hPG layer.

The biomarker expression level of different cancer cell lines greatly determines the cell-interface binding affinity.^{[42]} To explore the specificity of these nanostructured biointerfaces for cancer cell capture, three kinds of cell lines, i.e., MCF7 and T47D (high EpCAM expression), MDA-MB-231 and A549 (low EpCAM expression), GFP+-HeLa (EpCAM-negative cells), were spiked into serum-free medium and incubated with anti-EpCAM functionalized mPG4 surfaces for 1 hour, respectively. Similar to MCF7 cells, high EpCAM expression T47D displayed a higher affinity to the biofunctionalized surfaces and more than 88% of spiked cells were specifically captured onto anti-EpCAM-mPG4 surfaces. Even the MDA-MB-231 and A549 cells have a lower EpCAM expression on the cell membrane, there are still -75% of MDA-MB-231 cells and -80% A549 cells were successively isolated by these biointerfaces. In contrast, the antibody functionalized mPG4 surfaces exhibited a very low capture efficiency for EpCAM-negative HeLa cells (<5%), which may be nonspecifically trapped on surfaces. It needs to notice that the capture efficiency for both high- and low-EpCAM expression cancer cells were remarkably enhanced by the surficial nanostructured features, especially for the cell lines with lower EpCAM expression level. More than 5 times and 2 times capture efficiency improvements were found on the anti-EpCAM functionalized mPG4 surfaces compared to the smooth control surfaces for A549 cancer cells and MDA-MB-231 cancer cells, respectively.

To further verify the potential application of this nanostructured biointerface in cancer diagnosis, the cancer cells were then spiked into human blood to obtain artificial cancer patient's blood samples. As shown in Figure 4C, roughly 90% of MCF7 cancer cells were captured from lysed blood and ∼72% were captured from the whole blood sample by the nanostructured biointerface (anti-EpCAM-mPG4) after 1-hour incubation. Immunostaining was used to distinguish the captured cancer cells from blood cells on nanostructured surfaces. Captured cells were identified as CTC cells when they were immunostained positive for anti-CK18 (a protein marker for epithelial cells) but negative for anti-CD45 (CD45, a marker for leukocytes).

Compared with nanostructured surfaces without antifouling background and specific biomarker, the anti-EpCAM-mPG4 nanostructured surfaces displayed a high biospecificity for MCF7 cancer cells (CK18+/CD45-/DAPI+) and excellent resistance to the adhesion of leukocytes (CK18-/CD45+/DAPI+). The resulting capture purity for anti-EpCAM-mPG4 nanostructured surfaces was 93 ± 2.8%. It is known that the abundance of CTCs in patient blood samples is very rare and ranges from a few to hundreds per milliliter.^{[43]}

Thus, to explore the detection sensitivity of these nanostructured biointerfaces, MCF7 cancer cells were further spiked into lysed whole blood with a concentration from 2000 cells mL⁻¹ to 2 cells mL⁻¹. As shown in Figure 4D, a nice linear correlation between the number of spiked cells and captured cells was observed. These biospecific nanostructured interfaces can still efficiently capture the CTCs from a very dilute suspension, i.e., ∼2 cells mL⁻¹ (capture efficiency >75%).

### Nanostructured topographic features enhance antibody conjugation and cancer cell adhesion

To explore the underlying mechanism responding for the high cancer cell capture performance on nanostructured surfaces, immunochemistry was first performed to quantify the density of conjugated antibody on topographic surfaces with different surface roughness.

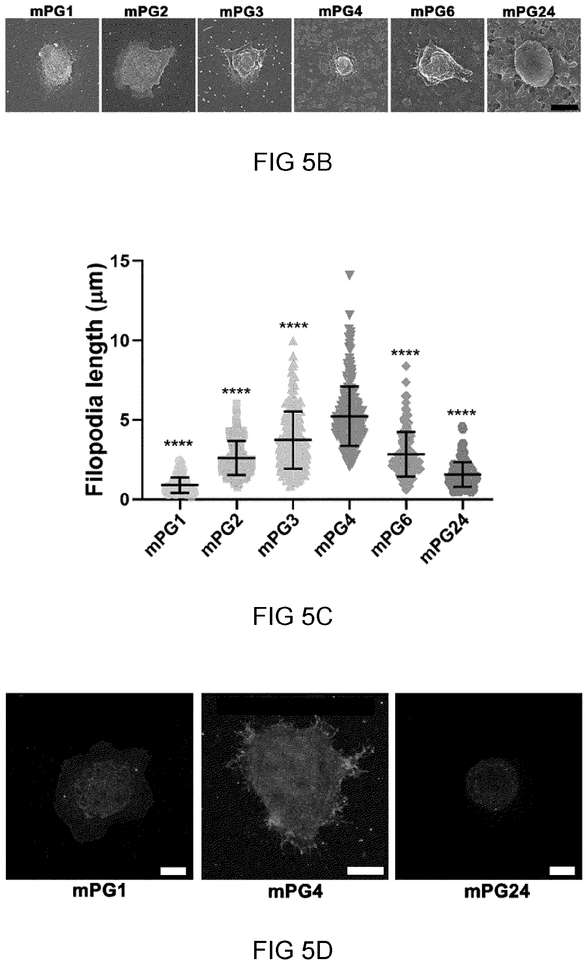

Figure 5A shows immunofluorescent images and the quantification of the relative conjugated antibodies on nanostructured surfaces with different surface roughness. The antibody conjugated surfaces were immunofluorescence stained by Cy5-conjugated goat polyclonal secondary antibody to mouse IgG. Figure 5B shows filopodia length of the adhered cancer cells on nanostructured surfaces and Figure 5C shows the representative SEM images of adhered cancer cells on nanostructured surfaces (n = 10-15, 3 technical replicates). The scale bar indicates 10 µm. Figure 5D shows representative fluorescence images of paxillin immunostaining cancer cells on mPG0, mPG4, and mPG24 surfaces, respectively. The scale bar indicates 10 µm.

As shown in Figure 5A, the antibody conjugation on the coating surface was progressively increasing with the increase of surface roughness, which provides more surface area for biomarkers immobilization. Then the detailed morphology of the captured cells on nanostructured surfaces with different roughness were observed by scanning electron microscope (Figure 5B). This indicates that the cancer cells prefer to spread on surfaces with lower roughness and maintained a round morphology. The spreading of the cells on nanostructured surfaces slightly decreased but more protruded with the increase of surface roughness. Filopodia are actin-rich plasma-membrane protrusions and function as antennae for cells to probe their environment,^{[44]} which play an important role in cell-interface interaction during cell adhesion and spreading. The length of the filopodia of the adhered cells on nanostructured surfaces was then quantified. Only a few and short filopodia were observed from cells on the surface with lower roughness (Figures 5B and 5C). But the filopodia formation on nanostructured surfaces was gradually enhanced with the increase of surface roughness and maximized at mPG4 surfaces. Thereafter, the increased roughness largely restricted the expression of filopodia on nanostructured surfaces. It means the topographical features would strengthen cell-biointerface interaction if their size were in a suitable range, i.e, < 350 nm for MCF7 cells. The further increased surface roughness possibly limits cell extension in the horizontal direction and the cells adapt themselves to minimize their contact with those surficial features.^{[3]} Accordingly, cell membrane mobility and filopodia formation are hindered during cell adhesion.^{[28]} Thus, this roughness selective manner of cell-interface interactions explains the roughness-dependent capture efficiency of cancer cells on nanostructured surfaces. It also demonstrates that the enhancement effect of surficial topographical features on cell capture should be in a preferred range according to the size of cells.^{[19, 22]}

Cell sense and respond to substrate topography through integrin-mediated mechanotransduction. Paxillin in adhesion complexes was stained to examine the effect of topographical features on focal adhesions (FAs) formation. As shown in Figure 5D, clear punctate structures and mature FAs in cells on mPG4 surfaces were observed, while no obvious punctate structures and FAs were found on the mPG1 surface with lower roughness (Rq = 56 nm) and the mPG24 surface with higher roughness (Rq = 572 nm). The molecular arrangement of integrin-mediated FAs formation is sensitive to the surficial nanostructured features but should be in a suitable range, and hence possibly results in the distinct adhesion behaviors.

Overall, the nanostructure features on biointerfaces could greatly increase the surface area available for bioligands conjugation and the contact between nanoscale cell-surface components and the nanofeatures on biointerfaces. Furthermore, the nanostructures that mimic the nanoscale features found in tissue microenvironment could enhance the formation of filopodia and focal adhesion, resulting in highly efficient affinity capture.

### Reversible release of captured cancer cells via dynamic ligands bonding.

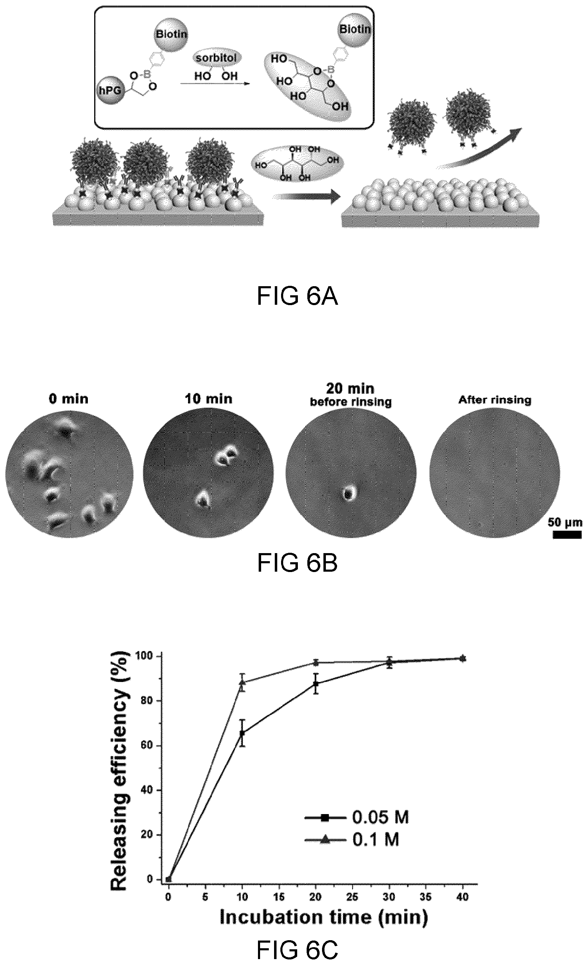

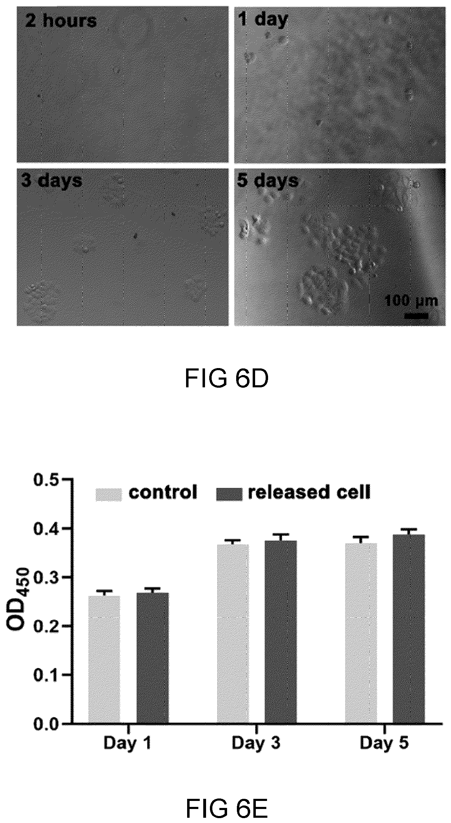

Figure 6A schematically illustrates the mechanism of sorbitol-induced cancer cell release. Sorbitol has a stronger affinity than the diols from hPG to PBA and results in the release of captured cells from the surface by competitive binding. Figure 6B shows incubation time and sorbitol concentration-dependent release efficiency of cancer cells on anti-EpCAM-mPG4 surfaces. Figure 6C shows representative microscopy images of cancer cell responsive release on mPG4(+) surfaces. Figure 6D shows the cell viability of released cancer cells from mPG4(+) surfaces by re-culturing. Figure 6E shows the proliferation analysis of the released cells by CCK-8 assay. The OD value at 450 nm represents the activity of cancer cells. The scale bar indicates 100 µm.

Benefiting from the dynamic cis-diols/PBA binding chemistry, the competitive dissociation of conjugated bioligands facilitates the responsive detachment of captured cells from the nanostructured surfaces in the presence of higher affinity cis-diols to phenylboronic acid, e.g., sorbitol^{[29]} (Figure 6A). After adding the glycan into the medium (0.1 M sorbitol), the spread cells on the nanostructured surfaces gradually round-up (Figure 6B). Both the cell morphology and adhesion status dramatically altered after 10 min incubation at 37 °C. Almost all the captured cells were easily removed by gently rinsing with PBS buffer after 20 min incubation (Figure 6B). This sugar-responsive cell detachment results from the competitive unbinding of bioligands from basal surfaces. Thus, cells cannot maintain adhesion onto antifouling surfaces anymore in the absence of adhesive ligands and subsequently detach. The sorbitol dose-dependent and incubation time-dependent releasing efficiency were also investigated and shown in Figure 6C. Although the detachment of cells under 0.1 M sorbitol solution was comparably quicker than that under 0.05 M sorbitol solution at the beginning, there was no significant difference on their release efficiency after 30 min incubation 98 ± 1.5% of release efficiency has already been achieved. In addition to the high detachment efficiency, the integrity and viability of the released cells are required and very important for downstream biological analysis. Thus, the released cells were re-cultured in a fresh medium and the proliferation ability of released cells was quantified via CCK8 assay (Figures 6D and 6E). Compared with the control group, the released cells still kept high viability, and no significant difference on the proliferation was observed between the control cells and the released cells even using a higher concentration of sorbitol.

### Conclusion

Overall, an advanced biointerface with well-defined topographical features was developed via a new generation of mussel-inspired coating polymers. The adhesion of cancer cells on these nanostructured biointerfaces was greatly enhanced compared with smooth surfaces and exhibited an interesting biphasic roughness-dependent manner. Under the presence of an antifouling coating layer and conjugated biospecific ligands, the generated biofunctional nanostructured surfaces exhibited a high performance for specific cancer cell capture that results from the improvement of bioligand conjugation, from an increase of the cell-biointerface contact area and an enhancement of filopodia formation and focal adhesion by the topographical features in an optimized size. It should be highlighted that the hPG monolayer on the biointerfaces herein not only provides an excellent bioinert background but also enables direct bioligand conjugation through the phenylboronic acid-diols dynamic binding and subsequently detach the bioligands via competitive glycan unbinding. Thus, the resulting surfaces are able to biospecifically capture circulating tumor cells with high efficiency (> 95%) and purity (> 96%) and "on-demand" release the captured cells quickly without damage. These results provide a new strategy to regulate cancer cell adhesion/capture via the biophysical cues and prospects for designing integrated biointerfaces for advanced cell-based biomedical studies in the future. Furthermore, the proposed method for *in-situ* conjugating and dissociating bioligands onto the bioinert surface via the responsive diols/PBA binding is not limited to the anti-EpCAM for cancer cell isolation but also works for versatile homo/hetero-bioligands immobilization once they have been modified with phenylboronic acid, which simplifies the multivalent and multifunctional biointerfaces preparation.

### List of references cited in the preceding sections

[1] A. Abbott, Nature 2003, 424, 870.
[2] Y. Zhu, Q. Zhang, X. Shi, D. Han, Adv. Mater. 2019, 31, 1804950.
[3] K. Anselme, L. Ploux, A. Ponche, J. Adhes. Sci. Technol. 2010, 24, 831.
[4] Z. Ma, M. Kotaki, R. Inai, S. Ramakrishna, Tissue Eng. 2005, 11, 101.
[5] A. Barradas, L. W. Terstappen, Cancers 2013, 5, 1619.
[6] M. Cristofanilli, G. T. Budd, M. J. Ellis, A. Stopeck, J. Matera, M. C. Miller, J. M. Reuben, G. V. Doyle, W. J. Allard, L. W. Terstappen, N. Engl. J. Med. 2004, 351, 781.
[7] T. Fehm, L. Morrison, H. Saboorian, L. Hynan, T. Tucker, J. Uhr, Breast Cancer Res. Treat. 2002, 75, 227.
[8] L. Carter, D. G. Rothwell, B. Mesquita, C. Smowton, H. S. Leong, F. Fernandez-Gutierrez, Y. Li, D. J. Burt, J. Antonello, C. J. Morrow, Nat. Med. 2017, 23, 114*.*
[9] C. Alix-Panabieres, K. Pantel, Nat. Rev. Cancer 2014, 14, 623.
[10] Y. Q. Li, B. K. Chandran, C. T. Lim, X. Chen, Adv. Sci. 2015, 2, 1500118.
[11] S. A. Joosse, T. M. Gorges, K. Pantel, EMBO Mol. Med. 2015, 7, 1.
[12] C. Alix-Panabieres, H. Schwarzenbach, K. Pantel, Annu. Rev. Med. 2012, 63, 199.
[13] B. J. Green, T. Saberi Safaei, A. Mepham, M. Labib, R. M. Mohamadi, S. O. Kelley, Angew. Chem., Int. Ed. 2016, 55, 1252.
[14] J. Chen, L. Yu, Y. Li, J. L. Cuellar-Camacho, Y. Chai, D. Li, Y. Li, H. Liu, L. Ou, W. Li, Adv. Funct. Mater. 2019, 29, 1808961.
[15] L. Wang, W. Asghar, U. Demirci, Y. Wan, Nano Today 2013, 8, 374.
[16] H. Cui, B. Wang, W. Wang, Y. Hao, C. Liu, K. Song, S. Zhang, S. Wang, ACS Appl. Mater. Interfaces 2018, 10, 19545.
[17] J. Meng, P. Zhang, F. Zhang, H. Liu, J. Fan, X. Liu, G. Yang, L. Jiang, S. Wang, ACS Nano 2015, 9, 9284.
[18] J. Dong, Y. J. Jan, J. Cheng, R. Y. Zhang, M. Meng, M. Smalley, P.-J. Chen, X. Tang, P. Tseng, L. Bao, Sci. Adv. 2019, 5, eaav9186.
[19] X. Liu, L. Chen, H. Liu, G. Yang, P. Zhang, D. Han, S. Wang, L. Jiang, NPG Asia Mater. 2013, 5, e63.
[20] D.-J. Kim, J.-K. Seol, G. Lee, G.-S. Kim, S.-K. Lee, Nanotechnology 2012, 23, 395102.
[21] S. Qi, C. Yi, S. Ji, C.-C. Fong, M. Yang, ACS Appl. Mater. Interfaces 2009, 1, 30.
[22] J. Dong, J. F. Chen, M. Smalley, M. Zhao, Z. Ke, Y. Zhu, H. R. Tseng, Adv. Mater. 2020, 32, 1903663.
[23] W. Chen, S. Weng, F. Zhang, S. Allen, X. Li, L. Bao, R. H. Lam, J. A. Macoska, S. D. Merajver, J. Fu, ACS nano 2013, 7, 566.
[24] F. Zhang, Y. Jiang, X. Liu, J. Meng, P. Zhang, H. Liu, G. Yang, G. Li, L. Jiang, L.-J. Wan, Nano Lett. 2016, 16, 766.
[25] Z. Zhang, N. Chen, S. Li, M. R. Battig, Y. Wang, J. Am. Chem. Soc. 2012, 134, 15716.
[26] H. E. Canavan, X. Cheng, D. J. Graham, B. D. Ratner, D. G. Castner, J. Biomed. Mater. Res. A 2005, 75, 1.
[27] Y. Hou, L. Yu, W. Xie, L. C. Camacho, M. Zhang, Z. Chu, Q. Wei, R. Haag, Nano Lett. 2019, 20, 748*.*
[28] Y. Hou, W. Xie, L. Yu, L. C. Camacho, C. Nie, M. Zhang, R. Haag, Q. Wei, Small 2020, 16, 1905422.
[29] G. Springsteen, B. Wang, Tetrahedron 2002, 58, 5291.
[30] Q. Wei, K. Achazi, H. Liebe, A. Schulz, P. L. M. Noeske, I. Grunwald, R. Haag, Angew. Chem., Int. Ed. 2014, 53, 11650.
[31] C. Schlaich, L. Cuellar Camacho, L. Yu, K. Achazi, Q. Wei, R. Haag, ACS Appl. Mater. Interfaces 2016, 8, 29117.
[32] M. J. La Voie, B. L. Ostaszewski, A. Weihofen, M. G. Schlossmacher, D. J. Selkoe, Nat. Med. 2005, 11, 1214.
[33] E. W. Danner, Y. Kan, M. U. Hammer, J. N. Israelachvili, J. H. Waite, Biochemistry 2012, 51, 6511.
[34] H. Lee, S. M. Dellatore, W. M. Miller, P. B. Messersmith, Science 2007, 318, 426.
[35] Q. Wei, F. Zhang, J. Li, B. Li, C. Zhao, Poly. Chem. 2010, 1, 1430.
[36] M. Yu, A. Bardia, B. S. Wittner, S. L. Stott, M. E. Smas, D. T. Ting, S. J. Isakoff, J. C. Ciciliano, M. N. Wells, A. M. Shah, Science 2013, 339, 580.
[37] F. Rusmini, Z. Zhong, J. Feijen, Biomacromolecules 2007, 8, 1775.
[38] W. A. Osta, Y. Chen, K. Mikhitarian, M. Mitas, M. Salem, Y. A. Hannun, D. J. Cole, W. E. Gillanders, Cancer Res. 2004, 64, 5818.
[39] S. Jeon, J. M. Moon, E. S. Lee, Y. H. Kim, Y. Cho, Angew. Chem. 2014, 126, 4685*.*
[40] L. Yang, H. Sun, W. Jiang, T. Xu, B. Song, R. Peng, L. Han, L. Jia, Chem. Mater. 2018, 30, 4372.
[41] X. Dou, P. Li, S. Jiang, H. Bayat, H. Schönherr, ACS Appl. Mater. Interfaces 2017, 9, 8508.
[42] E. Sollier, D. E. Go, J. Che, D. R. Gossett, S. O'Byrne, W. M. Weaver, N. Kummer, M. Rettig, J. Goldman, N. Nickols, Lab Chip 2014, 14, 63.
[43] A. van de Stolpe, K. Pantel, S. Sleijfer, L. W. Terstappen, J. M. Den Toonder, Cancer Res. 2011, 71, 5955*.*
[44] P. K. Mattila, P. Lappalainen, Nat. Rev. Mol. Cell Biol. 2008, 9, 446*.*
[45] A. Sunder, R. Mülhaupt, R. Haag, H. Frey, Adv. Mater. 2000, 12, 235.
[46] A. Sunder, R. Hanselmann, H. Frey, R. Mülhaupt, Macromolecules 1999, 32, 4240.

## Claims

1. Layered structure, comprising
a) a base layer of nanoparticles of crosslinked molecules of a linear polyglycerol derivative, wherein the linear polyglycerol derivative has at least one first terminal catechol group,
b) a coating layer comprising a hyperbranched polyglycerol derivative having at least one second terminal catechol group, wherein the hyperbranched polyglycerol derivative is covalently bound via the second terminal catechol group to the nanoparticles of the base layer, and
c) a ligand layer comprising a ligand covalently bound to the hyperbranched polyglycerol derivative of the coating layer, wherein the ligand is capable of non-covalently bonding an antibody.

2. Layered structure according to claim 1, **characterized in that** the base layer has a surface roughness lying in a range of from 1 nm to 1 µm.

3. Layered structure according to claim 1 or 2, **characterized in that** the linear polyglycerol derivative has a structure corresponding to general formula (I): 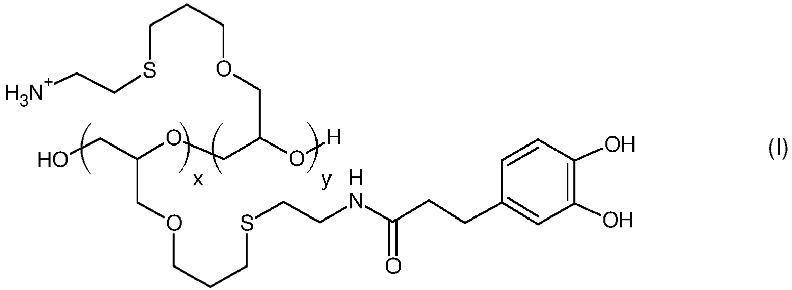 wherein
x is 1 to 1000 and
y is 1 to 1000.

4. Layered structure according to any of the preceding claims, **characterized in that** the ligand is covalently bond to the hyperbranched polyglycerol derivative via a phenylboronic ester bond.

5. Layered structure according to any of the preceding claims, **characterized in that** the ligand is a phenylboronic-acid modified biotin.

6. Layered structure according to any of the preceding claims, **characterized in that** the layered structure further comprises an antibody non-covalently bound to the ligand.

7. Layered structure according to claim 6, **characterized in that** the antibody is bound to the ligand via an avidin linker.

8. Biointerface assembly, comprising a substrate and a layered structure according to any of the preceding claims having a base layer of nanoparticles of crosslinked molecules of a linear polyglycerol derivative, wherein the linear polyglycerol derivative has at least one first terminal catechol group, wherein the nanoparticles of the layered structure are covalently bound to the substrate via the first terminal catechol group.

9. Biointerface assembly according to claim 8, **characterized in that** the substrate comprises at least one of magnetic nanoparticles, glass, polycarbonate, poly(methyl methacrylate), and polystyrene.

10. Method for manufacturing a layered structure according to any of claims 1 to 7, comprising the following steps:
a) allowing a crosslinking and an aggregation of a linear polyglycerol derivative in a basic aqueous buffer, wherein the linear polyglycerol derivative has at least one first terminal catechol group, so as to form nanoparticles of the linear polyglycerol derivative, the nanoparticles representing a base layer,
b) applying a coating layer onto the nanoparticles, wherein the coating layer comprises a hyperbranched polyglycerol derivative having at least one second terminal catechol group, wherein the hyperbranched polyglycerol derivative is covalently bound via the second terminal catechol group to the nanoparticles of the base layer,
c) forming a ligand layer on top of the coating layer, the ligand layer comprising a ligand covalently bound to the hyperbranched polyglycerol derivative of the coating layer, wherein the ligand is capable of non-covalently bonding an antibody.

11. Use of a layered structure according to any of claims 1 to 7 or a biointerface assembly according to claim 8 or 9 for capturing cells in vitro.

12. Use according to claim 11, **characterized in that** the cells are circulating tumor cells.

13. Layered structure according to any of claims 1 to 7 or a biointerface assembly according to claim 8 or 9 for use in in-vivo diagnostics or therapy.

14. Layered structure according to any of claims 1 to 7 or a biointerface assembly according to claim 8 or 9 for use in in-vivo diagnostics or therapy of a cancer disease.

15. Method for capturing and optionally releasing cells in vitro, comprising the following steps:
a) providing a layered structure according to any of claims 1 to 7 or a biointerface assembly according to claim 8 or 9 with such a layered structure, wherein the layered structure comprises a base layer, a coating layer covalently bonded to the base layer, and an antibody non-covalently bound to the ligand via an avidin linker,
b) bringing the layered structure in contact with a body fluid sample, thereby allowing capturing of cells out of the body fluid sample by the antibody, and
c) optionally releasing the cells together with the bound antibody by rinsing the layered structure with a solution comprising a cis-1,2-diol.
